# EUROPEAN PATENT APPLICATION

(11) **EP 2 997 995 A2**
(43) Date of publication of application: **23.03.2016**
(21) Application number: 15185448.6
(22) Date of filing: 16.09.2015
(51) Int. Cl.: A61N 1/04, A61B 5/0478, F16G 11/10

(54) **CRANIAL POSITION DETERMINATION SYSTEM**

(30) Priority: 16.09.2014 US 201414487994
(71) Applicant: Nuraleve Inc., Ottawa, ON K2B 7G1 (CA)
(72) Inventor: WILLIAMS, Patrick, Chelsea, QC J9B 2H4 (CA); BAILEY, Kevin, Ottawa, ON K1Z 5M4 (CA); BLAIS, Crystal, Kanata, ON K2L 4G6 (CA); GARDIN, Paul, Ottawa, ON K1S 2H4 (CA)
(74) Representative: Bartle, Robin Jonathan

(57) **Abstract**

An electrode placement system has a headband that is adjustable for size two or more retainers slidably mounted on the headband and a first elastic measurement cord, wherein the cord is releasably held by the two or more retainers in order to determine a position on a cranium. An electrode retention system has a headband that is adjustable for size, having a plurality of attachment points thereon, two or more strips, each strip having two ends, wherein each strip is connected to an attachment point at one end, and at least one adjustable electrode holder configured to hold an electrode to a cranium, wherein first and second strips are connected to attachment points, and first and second strips pass through the electrode holder, and are independently retained by the adjustable electrode holder such that the electrode holder is adjustable for tension and position.

## Description

### Field

The invention relates to the field of systems to determine relative positions on the cranium.

### Background

When administering treatments involving tDCS, tACS, EEG, TMS or other application of electrical stimulation to a patient's cranium, correct electrode placement must be determined in advance of treatment, so that the correct region of the brain is stimulated. Electrodes positioning is not an exact science, as each cranium is slightly different, however there are recognized methods that are followed by professionals administering such treatment, primarily including the International 10-20 system and International 10-10 system. In these systems, locations on the scalp are found in a repeatable manner by measuring distances between anatomical landmarks - such as the nasion, inion and earlobes - and marking locations at a fixed percentage of the distance between them.

In the past, these measurements were taken by a nurse or other practitioner who would measure the head and calculate distances. In one example, a practitioner would locate the F3/F4 regions of the International 10-20 system on a patient as follows: if the distance between the nasion and the inion is 13", division by two reveals a midpoint of 6.5", which is marked on the scalp. Next, the distance from earlobe to earlobe over the top of the head, passing through the marked nasion/inion midpoint was measured. A point was marked along the measurement line between the nasion/inion midpoint to the earlobe on each side. This point was marked 20% of the way down from the midpoint moving towards the earlobe. A forward measurement of 5cm was applied to each 20% point to determine each of two electrode positions on the cranium, at the F3/F4 positions.

This process of measurement by medical personnel is very time consuming for patient and medical practitioner, and can lead to errors in calculation with resulting errors in electrode positioning. The effects of an error may range from merely ineffective treatment to possible harm to the patient, depending on the parameters of the treatment that is applied and the sensitivity of the patient.

Once electrode positions are determined, the electrodes must be held against the patient's cranium in the proper position and with a small amount of force, such that the electrode is held but the pressure does not become uncomfortable to the patient. Prior art reveals various means to hold the electrodes to the head, including rubber balaclavas with holes for the electrodes at appropriate positions. However, such devices may be uncomfortable to wear for any period of time, are difficult to don without disrupting electrode placement, and would require a variety of sizing to accommodate different sizes of cranium. Other prior art includes elastic straps across the head that require two medical personnel to attach correctly.

Therefore there is a need in the art for a measurement apparatus and technique to facilitate accurate measurement of specific points on the cranium for treatment, in order to reduce the time and effort required to hold the electrodes. In addition, there is a need in the art for a means, administrable by a single person, to quickly place the electrodes on the measured sites and apply sufficient pressure to hold the electrodes comfortably for treatment that is adjustable to fit the majority of patients without the need for different sizes.

### Summary

An electrode placement system has a headband that is adjustable for size two or more retainers slidably mounted on the headband and a first elastic measurement cord, wherein the measurement cord is releasably held by the two or more retainers in order to determine a position on a cranium.

In one embodiment a second measurement cord is affixed perpendicularly to the first measurement cord. In another embodiment the measurement cords have distance markings thereon. Furthermore, flags may be affixed to the measurement cord, configured to measure a distance perpendicularly from the measurement cord. The first measurement cord may measure from a neck to a nose of a patient, and the second measurement cord measures from ear to ear of the patient.

In one embodiment the retainers comprise a frame defining a restriction for a cord and a cam in rotating relation with the frame having a gripping edge, wherein when the cam is rotated in a tightening direction, the cam protrudes into the restriction, and wherein when the cam is rotated in a loosening direction, the cam recedes from the restriction, and wherein the gripping edge is configured to engage with the cord to rotate the cam.

An electrode retention system has a headband that is adjustable for size, having a plurality of attachment points thereon, two or more strips, each strip having two ends, wherein each strip is connected to an attachment point at one end, and at least one adjustable electrode holder configured to hold an electrode to a cranium, wherein first and second strips are connected to attachment points on the headband, and first and second strips pass through the at least one adjustable electrode holder, and the first and second strips are independently retained by the adjustable electrode holder such that the at least one electrode holder is adjustable for tension and position.

In one embodiment the headband is adjustable by an adjustment means, the adjustment means comprising toothed tabs extending from ends of the headband, and a rotatable gear in communication with the toothed tabs, wherein when the gear is rotated in a tightening direction it actuates the toothed tabs in opposite directions, tightening the headband.

In one embodiment the attachment points comprise inverted hooks having apertures therebelow, wherein the end of each strap has an eyelet, and the eyelet is configured to engage and disengage with the hook while the strap is in a horizontal orientation, and to be locked on the hook when the strap is in a vertical orientation.

In an embodiment the adjustable electrode holder comprises a housing through which the strips pass a gear cluster in the housing, comprising a control gear rotatably mounted on the housing by a displaceable axle, a first drive gear rotatably mounted to the housing and in communication with the first strip, a second drive gear rotatably mounted to the housing and in communication with the second strip, wherein the strips have teeth for engaging with the drive gear teeth, and wherein in a tension adjustment position the first and second drive gears are connected by the control gear, and in a position adjustment position the control gear is displaced away from the drive gears, and the drive gears are connected directly to one another.

A button may be present on the housing for selecting a tension adjustment position and a position adjustment position, wherein the control gear is biased in a tension adjustment position. Furthermore, the adjustable electrode holder may have a housing through which the strips pass, two releasable locks mounted to the housing, wherein a strip passes through each releasable lock, a button for releasing each releasable lock, wherein while the button is held the strip is movable within the releasable locks and when the button is released the strip is locked within the releasable lock.

In one embodiment each of the buttons for a releasable lock are opposite one another and are configured to be depressed by one hand. The electrode retention system may have projections opposite the buttons for bracing to depress the button with one hand.

### Description of Figures

Figure 1a shows an adjustable headband, according to an embodiment of the present invention;
Figure 1b shows the headband mounted on a patient's cranium, according to an embodiment of the present invention;
Figure 1c shows a detail view of the headband adjustment mechanism, according to an embodiment of the present invention;
Figure 2 shows the measurement subsystem, according to an embodiment of the present invention;
Figure 3 shows the headband mounted on a patient's cranium with the measurement subsystem, according to an embodiment of the present invention;
Figure 4a shows an embodiment of a camming retainer, according to one embodiment of the present invention;
Figure 4b shows an embodiment of a camming retainer with a longitudinal cord inserted, according to one embodiment of the present invention;
Figure 4c shows an embodiment of a camming retainer with the cam removed, according to one embodiment of the present invention;
Figure 4d shows detail view of a headband hook and eyelet, in one embodiment of the present invention;
Figure 5a shows a transparent view of an adjustable electrode holder in a tension adjustment position, according to one embodiment of the present invention;
Figure 5b shows a transparent view of an adjustable electrode holder in a position adjustment position, according to one embodiment of the present invention;
Figure 5c shows a further cutaway view of an adjustable electrode holder in a tension adjustment position, according to one embodiment of the present invention;
Figure 5d shows a cutaway view of the bezel on an adjustable electrode holder, according to one embodiment of the present invention;
Figure 6a shows a cut-away view of a gear arrangement in a tension adjustment position, according to one embodiment of the present invention;
Figure 6b shows a cut-away view of a gear arrangement in a position adjustment position, according to one embodiment of the present invention; and
Figure 7 shows an adjustable electrode holder, in a further embodiment of the present invention.

### Detailed Description

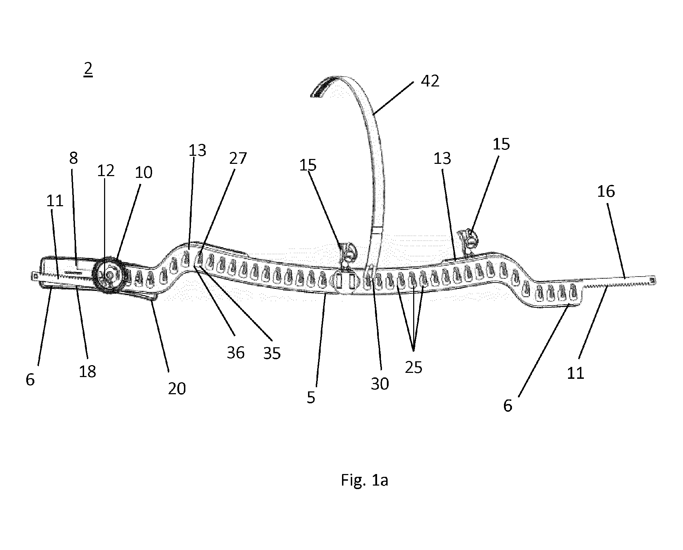

With reference to Figure 1a, the headband 2 is shown, outstretched. The headband has a band 5 that passes around the circumference of a patient's head. The band 5 is fastened together at the ends 6 and adjustable for size by means of an adjusting assembly having a gear 12 and two tabs 16, 18. The adjusting assembly has a rotating bezel 10 having a gear 12 on its backside. To facilitate the movement of the adjusting assembly without contacting the patient's head, a guard 8 has a pin 14 mounted thereon for engagement with the bezel 10. The bezel 10 is knurled for grip, and rotates either way on a pin 14 mounted to the guard 8. Other ways of adjusting the headband size are known in the art and may be used. Each end 6 of the band 5 is extended by a toothed tab 16, 18 having a width less than half of the width of the band and having teeth 11 for engaging with the gear 12. The band 5 has a track 13 along its upper edge along the partial or full length of the band 5. Within the track 13 retention means 15 are slidably mounted, wherein the retention means 15 are for retaining the appendages of the measurement web (not shown). Examples of retention means 15 are clips, hooks, clamps and other ways known in the art of slidably holding straps on the band. Further, spaced along the band 5 are inverted hooks 27 for holding straps 42.

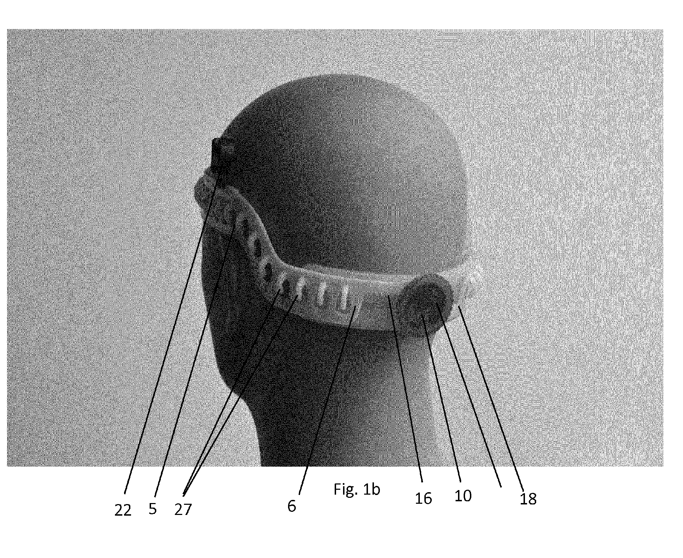

With reference to Figures 1a and 1b showing the headband closed and mounted on a patient's head, the toothed tabs 16, 18 extend from the ends 6 of the band 5 in opposite orientation to each other, such that the upper tab 16 passes over the gear 12, while lower tab 18 passes under the gear, wherein the toothed portion of each engages with the toothed gear. The tabs are held in contact with the toothed gear by a guide 20 on the guard 8. The tabs 16, 18 pass over the guard 8 and engage with the gear 12 mounted thereon. As the tabs move back and forth due to rotation from the gear 12, the patient's head is protected by the guard 8.

When the gear 12 is turned clockwise, it pulls the top tab 16 in and the bottom tab 18 in such a way that the tabs, and therefore the ends, are drawn towards one another wherein the circumference of the band 5 is reduced. When the gear 12 is turned counter-clockwise, it pushes the upper tab 16 out while also pushing the lower tab 18 out, such that the ends are pushed away from one another and the circumference of the band 5 increases.

With reference to Figure 1b, in an embodiment, the bezel 10 is a lockable bezel that comprises a release, wherein when the bezel 10 is locked, force from the toothed tabs 16, 18 in contact with the gear behind the bezel do not rotate the gear 12 (not shown, positioned behind the bezel 10). Once the bezel 10 is partially rotated, the release releases the lock 12a and the gear 12 is able to turn.

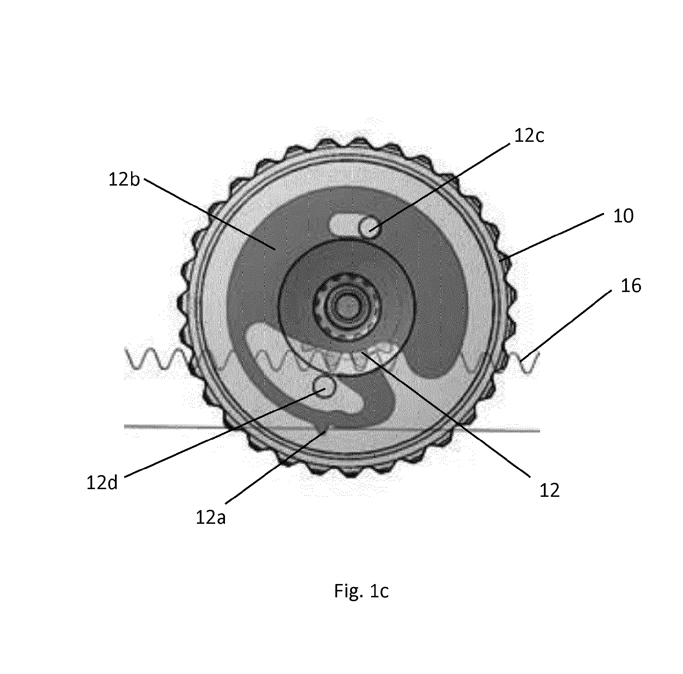

With reference to Figure 1c, the lockable bezel is shown. The gear 12 is fixedly attached to the ratchet spinner 12b whose spring tooth 12a is normally engaged into the circular rack (not shown) of the bezel 10 such that it can spin one way (tightening) but not the other (loosening) when driven by the gear 12. The adjustment knob has 2 posts 12c and 12d that go through the ratchet spinner 12b, wherein post 12c limits relative motion to a few degrees and post 12d engages to release the spring tooth 12a of the ratchet spinner 12b when the bezel 10 is turned in a loosening direction so that the tabs 16, 18 may be loosened. In loosening, post 12d travels up a ramp, pulling in the tooth 12a and disengaging it from the bezel teeth (not shown). In this way the headband 5 can be easily loosened and tightened using the bezel 10, but tightness in the band applying force to the gear 12 cannot loosen the ratchet spinner 12b when the bezel is locked.

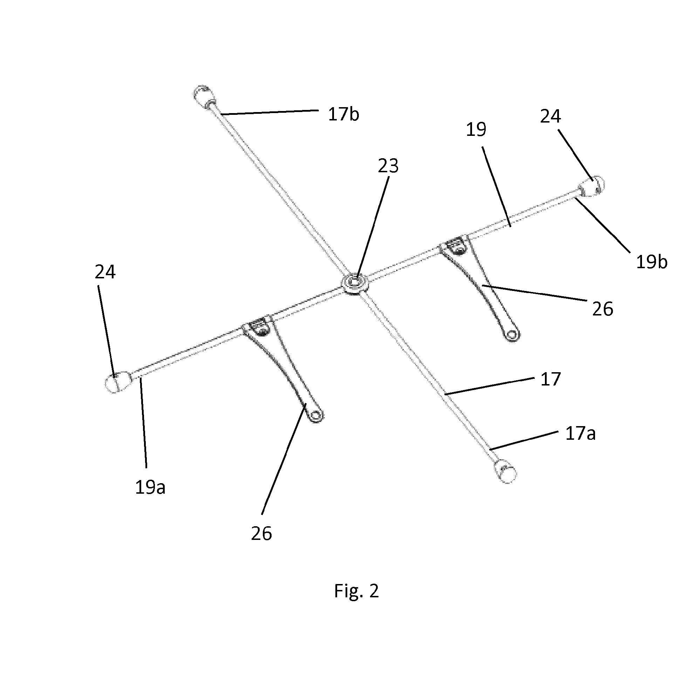

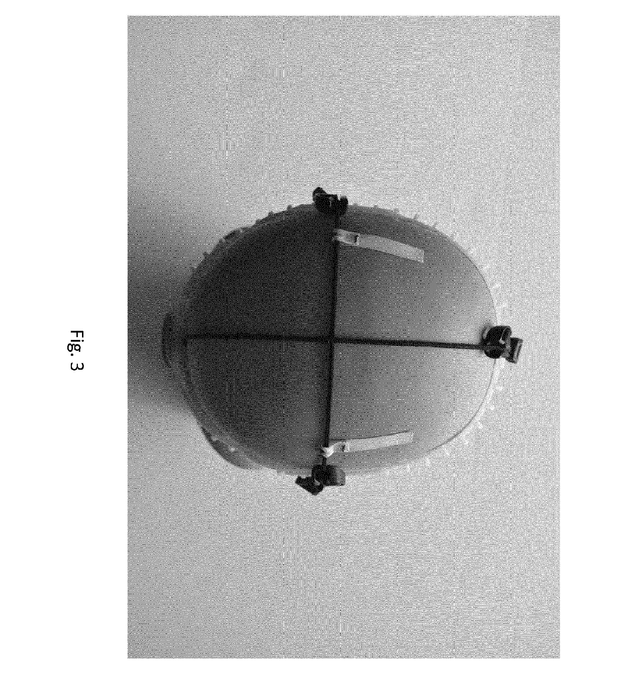

With reference to Figures 2 and 3 the measurement web is mounted to the rear of the band 5, in the nape of the neck area. It comprises two elastic cords, a longitudinal cord 17 and a transverse cord 19, in cruciate relationship to one another and affixed to one another at a midpoint 23. The longitudinal cord stretches from the nape of the neck to the bridge of the nose, and is retained at each end by retainer 22 mounted to the band 5. The nape end 17a of the longitudinal cord 17 is fixed at the nape of the neck, and the bridge end 17b may pulled across the head and retained in retainer 22 near the bridge of the nose. The transverse cord 19 stretches from the ear or sideburn area of the head, across the top of the head, to the other ear or sideburn area, and is retained at each side with retainer 22 mounted to the band 5. Each cord end 17a, 17b and 19a, 19b may have a plug thereon to facilitate holding and retention of the cord end. Further, the cords may have removable or permanently attached flags 26 of certain length to facilitate position determination, perpendicular to the cord on which they are attached.

The band 5 has a track 21 along its upper edge along the partial or full length of the band 5. Within the track 21 one or more retainers 22 for retaining the appendages of the measurement web are slidably mounted. The retainer holds the stretchable cord 17, 19 wherever it is introduced to the retainer, clamping the cord 17, 19 along its length or retaining the plug on the end of the cord 17, 19. For example, the cord 17, 19 will be stretched more in relation to a larger cranium than a smaller cranium, and in each case the ends of the cords are releasably held in the retainer 22. The cords 17, 19 may be marked with percentages, such as 20%, 40%, 60% representing 20%, 40% and 60% of the length respectively. The bands stretch uniformly so the percentage markings are always correct relative to the stretched length of the cords 17, 19, when the end of the cord is held by retainer 22. This enables a medical practitioner to observe positions on the head of a patient, based on the cruciate cords each marked with percentages. Starting at the midpoint on top of the head, the transverse cord each provide gradations of percentages to the ear on each side, and the longitudinal cord provides gradations to the nape of the neck, and to the bridge of the nose. Using this grid, the practitioner is able to accurately determine a position on the cranium, for electrode stimulation and other medical procedures.

In an example for treatment using tDCS and tACS, the position for each electrode is determined by following the transverse cord towards each ear to the 20% of the ear-to-ear distance (across the top of the head), and measuring directly forward (towards the patient's face) 5cm. Other treatments may use other locations, for treating or identifying other areas of the cranium and nervous tissue. Therefore the position of flags on the cords, and the length of each flag, may vary according to the intended treatment.

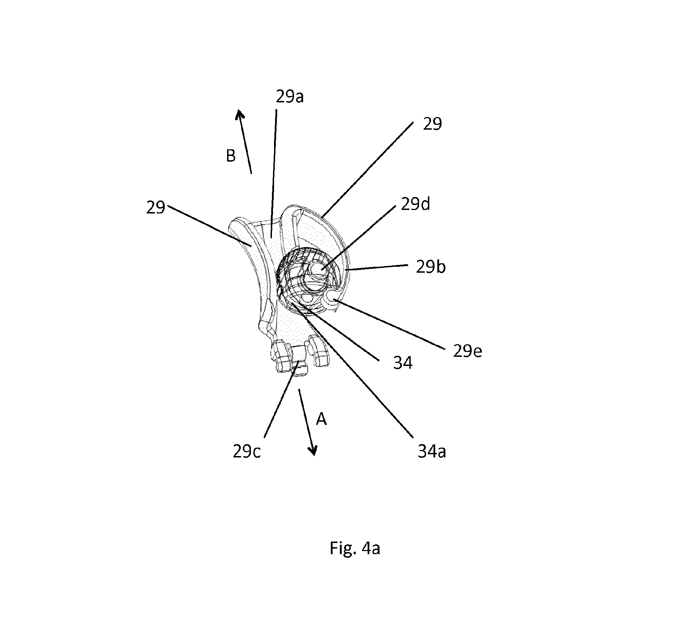

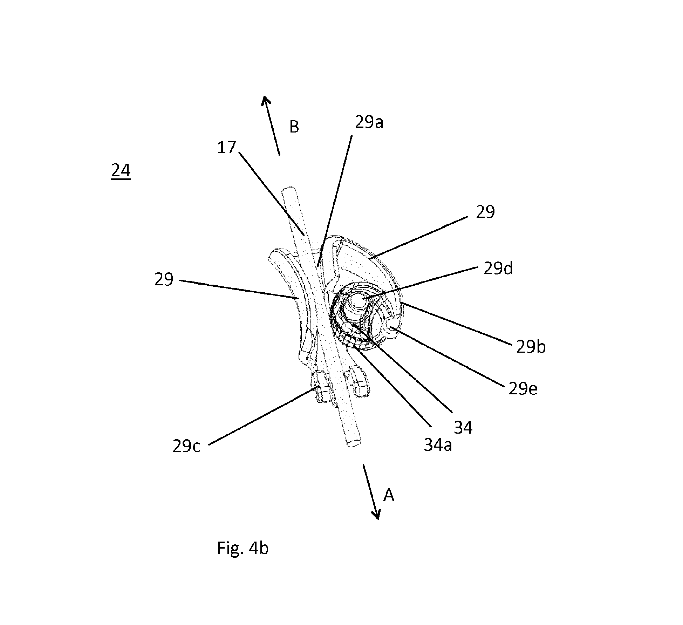

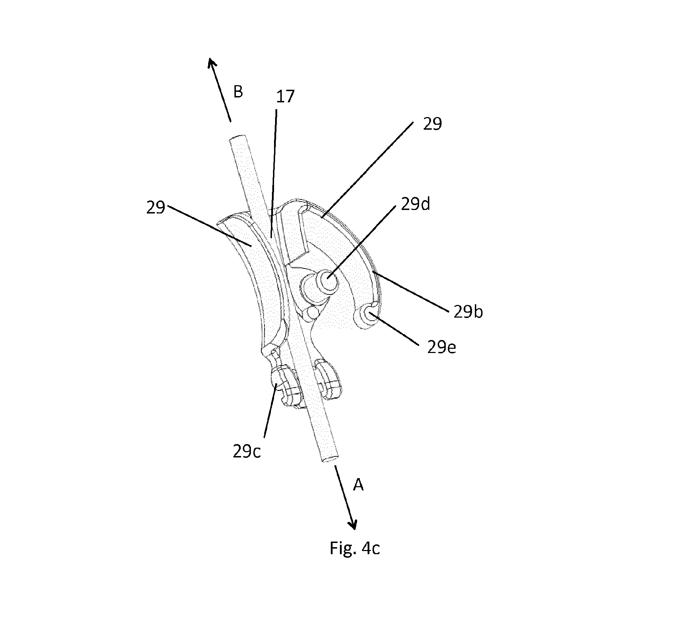

With reference to Figures 4a, 4b and 4c, in one embodiment the retainer 22 is a camming unit 24, which clamps the cord 17 or 19 (shown in Figure 4b) as tension is applied by the elastic cord and the cam 34 is rotated. The camming unit 24 has a frame 29 and restriction 29a through which the cord passes. The frame 29 has an arm 29b for holding the cam 34 onto the pivot pin 29d, by engaging with an indent on the face of the cam 34. The arm 29b acts as a light spring to force the cam 34 inwards to catch the cord 17 or 19. The frame 29 also has one or more mountingtabs 29c for mounting on the band 5. The frame 29 has a pin 29d for enabling a cam 34, mounted thereon, to rotate. A pivoting cam 34 with a gripping edge such as a knurled edge 34a, is positioned within the frame 29, such that it contacts the stop 29e to prevent free rotation of the cam 34. The cam may rotate in a restrictive direction, or a loosening direction. When the cord 17 or 19 is pulled through the camming unit in direction A (loosening direction), the cam 34 is rotated so it protrudes less into the restriction 29a; pressure is released and the cord may be removed or adjusted in a low-friction or near-frictionless manner. Conversely, in direction B (restrictive direction), the cam is shaped to narrow the restriction 29a, and compress the cord 17, 19, as it rotates. Once the cam 34 is in contact with the cord 17, 19, the gripping edge engages with the cord 17, 19, and a pull on the cord 17, 19 pivoting the cam 34 in restrictive direction B will increase lateral pressure on the cord 17, 19, inhibiting movement thereof. In other embodiments the retainers 22 are clamps, clips and fasteners known in the art for holding a cord.

In another embodiment, the retainer 22 may consist of cleats (not shown) or clamps (not shown) that hold the cord 17, 19.

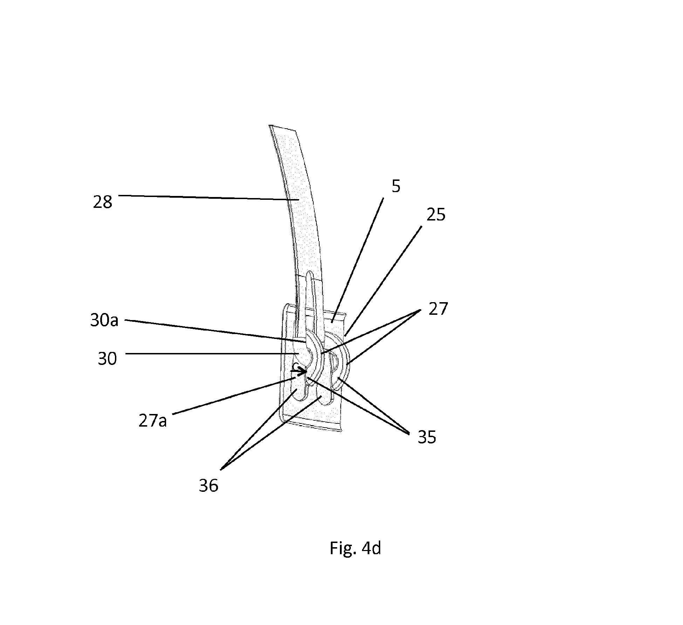

With reference to Figure 4d, the band 5 has a number of attachment points 25 along its length. The points 25 may be spaced evenly across the band 5 or may be concentrated in key positions. In an embodiment the attachment points are inverted hooks 27 which enable the mounting of overhead straps 28 having eyelets 30 at each end. Each eyelet 30 fits over a hook 27.

With reference to Figures 1a, 1b and 4d, in an embodiment, the hook opening 27a (distance C) is narrower than the width of the eyelet 30 when viewed flush to the band, as in Figure 4d, such that the eyelet 30 cannot exit the hook 27 when the strap 28 is vertically oriented. An aperture 36 is located below the hook 27, and the aperture 36 in combination with the hook opening 27a provides sufficient clearance for the eyelet 30 to pass through and mount on the hook 27 while the eyelet 30 is in a horizontal orientation, since the curvature 30a of the eyelet 30 causes some of the eyelet width to project into the aperture 36. Therefore, the eyelet 30 may be mounted on the hook 27 when the strap 28 is in a horizontal inclination. The eyelet 30 has a curvature 30a that enables it, when approaching the hook 27 horizontally, to pass below the surface of the band 5 through aperture 36 and engage the hook 27. Once engaged on the hook 27, and the eyelet 30 pulled upwards, the eyelet 30 cannot be pulled off the hook 2 7 while the strap 28 is vertically oriented, as it is held on the hook 27, and there is insufficient space between the end of the hook 27 and the band 5 for the eyelet 30 to pass through. It is only when the eyelet 30 is oriented horizontally, such that the curvature of the eyelet 30 enables it to project into the aperture 36 and pass over the tip 35 of the hook, that the eyelet and strap 28 may be removed from the hook 27.

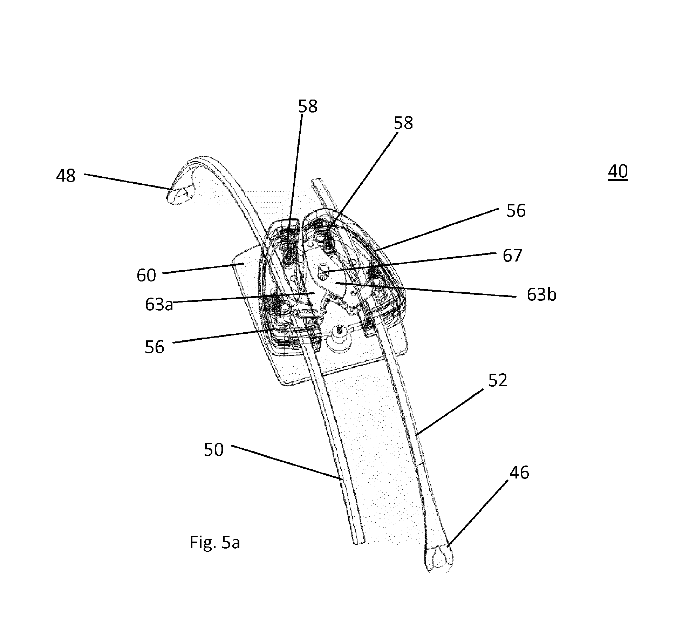

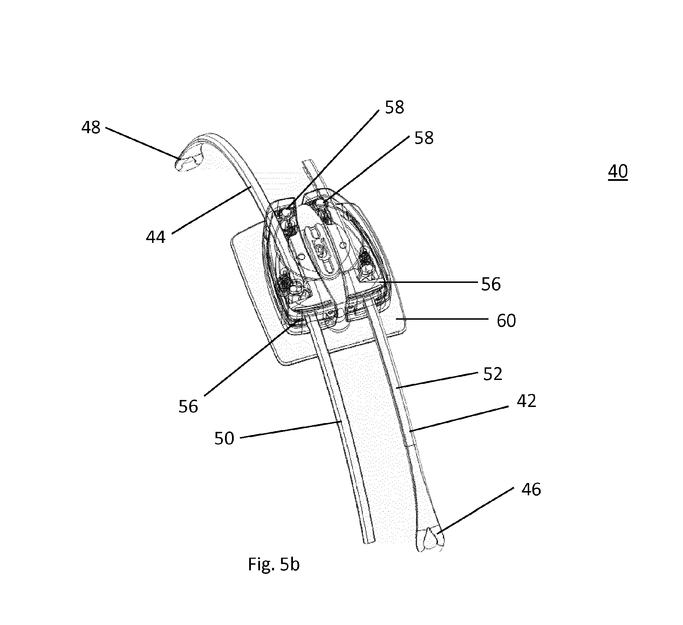

Figure 5a shows a transparent view of an adjustable electrode holder in a tension adjustment position, while Figure 5b shows the adjustable electrode holder in a position adjustment position. With reference to Figures 5a and 5b, an adjustable electrode holder 40 is shown, which is for passing over the cranium and for mounting to the headband 2. The electrode holder 40 has two teethed strips 42 and 44, each strip having an attachment ends 46, 48 for engaging with an attachment means 25 (not shown), and an opposite terminating end 50, 52 that simply terminates, such that it cannot be removed from the holder. The ends 46, 48 may have an eyelet 30 for attaching to the hook 27. The ends 46, 48 are oriented in opposition to one another such that the end 46 is fastened to the attachment means 25 (not shown) at one side of the headband 2 (not shown), and the end 48 is fastened to attachment means 25 (not shown) at the opposite side of the headband 2 (not shown), wherein each strip 42, 44 passes over the cranium.

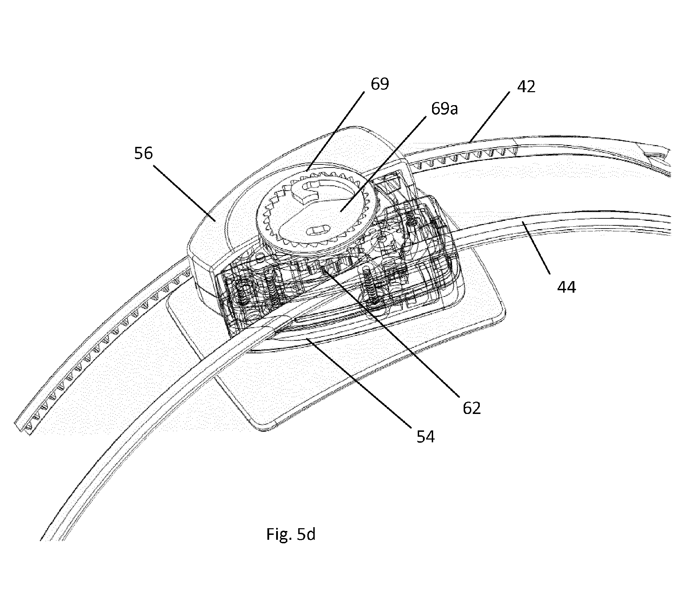

With reference to Figure 5d as well, each of the strips 42 and 44 passes through the housing 54, and has inward-facing teeth 55 for interfacing with gear teeth. The housing 54 has an electrode receiving pad 60 mounted thereto for receiving an electrode (not shown). Each side of the housing 54 has a button 56 which compresses inwardly into the housing, so that both buttons 56 may be pressed by squeezing toward each other with the thumb and forefinger. The buttons 56 are hinged to the housing 54 by a pin 58 on which they pivot. The buttons 56 are in contact with the strips 42, 44 and pressure on the buttons 56 will bias the strips 42, 44 inwardly. The buttons 56 are biased outwardly by a spring or otherwise, and require force to compress into the housing 54. Further, the buttons 56 will return to their original position once released.

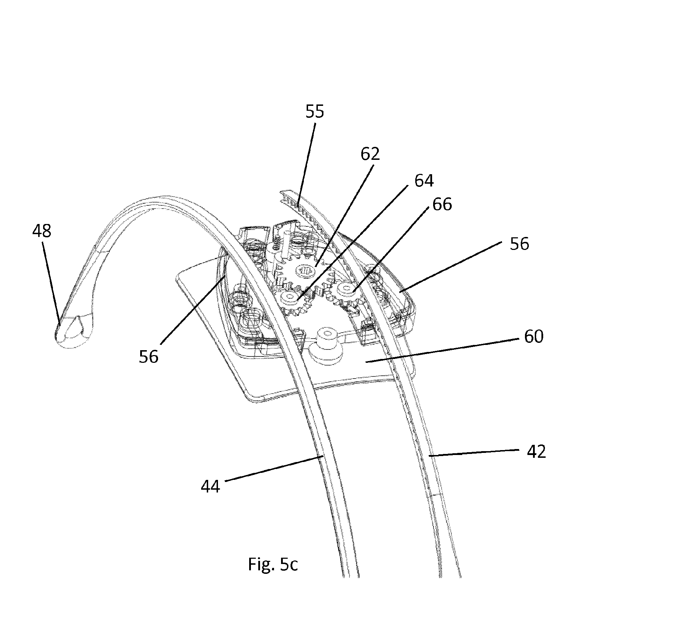

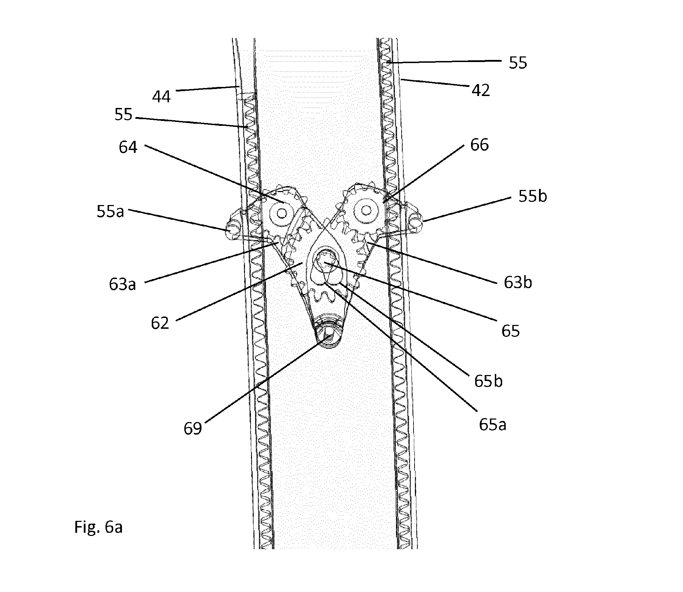

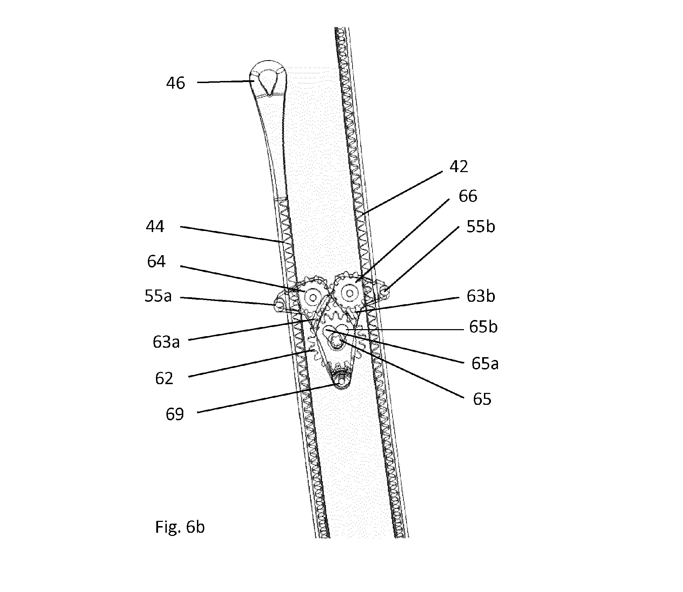

With reference to Figure 5c, the gear cluster within the housing 54 is shown. The buttons 56 push the drive gears 64, 66 together and control the engagement of the control gear 62, as seen in Figures 6a and 6b, below. The housing sits on an electrode receiving pad 60 that prevents hair or other foreign objects from entering the gear cluster.

With reference to Figure 5d, the control gear is actuated by a bezel 69. The axle 65 (not shown) of the control gear 62 extends through the housing and is fixed to a ratchet spinner 69a. The bezel 69 the spinner 69a ratchets against is slidingly mounted to the housing 54 between the buttons 56, so that it cannot spin with the spinner, but can slide back following the control gear 62 when the buttons are pressed and the control gear 62 is retracted. The spinner 69a in its bezel 69 prevents the strips 42, 44 from being pulled apart, but again, the bezel 69 can either tighten the spinner 69a, or when turned in a loosening direction, release the spinner 69a and loosen the assembly.

Figure 6a shows the gear cluster in a tension adjustment position, and Figure 6b shows the gear cluster in a position adjustment position. With reference to Figure 6a and 6b, a cluster of three spur gears is positioned within the housing 54. The two drive gears 64, 66 are mounted to two arms 63a, 63b that are hinged together behind the control gear 62 by pivot 69. The control gear 62 rotates on an axle 65, and is positioned by its axle 65, which passes through slots 65a, 65b respectively on arms 63a, 63b. As the arms 63a, 63b pivot closed to engage the drive gears together, the slots cross each other so as to drive the control gear 62 out of mesh or alignment with the drive gears 64,66. The control gear 62 is rotatably engaged with two smaller drive gears 64, 66 that respectively engage the teeth 55 of strips 42, 44. Strips 42, 44 are held against teeth 55 by guards 55a, 55b. The smaller drive gears 64,66 are outwardly biased, such that the drive gears are not in contact with one another in a resting, or tension adjustment, position. When the drive gears are pushed together into a position adjustment position, they mesh or align with each other and bypass the control gear 62. When the drive gears are pushed together, the control gear 62 moves up and away from the intersection of the drive gears 64, 66 along slots 67.

In an embodiment the drive gears are biased in a tension adjustment position to prevent unintentional movement of the housing while in position on a cranium. With reference to Figure 6a showing the tension adjustment position, while the buttons 56 are not depressed the drive gears 64, 66 are in contact with the control gear 62 and not with each other. Accordingly, the movement of the drive gears 64, 66 is controlled through movement of the control gear 62, with the result that the strips 42, 44 move in opposite directions relative to the housing 54. The strips 42, 44 both move into the housing 54 or out of the housing, so as to tighten or slacken the strips 42, 44 respectively by moving the attachment ends further apart or closer together.

With reference to Figure 6b showing the position adjustment position, when the buttons 56 are depressed, the drive gears 64, 66 and strips 42, 44 with which they are engaged are pushed together, bypassing the control gear 62 that is disengaged in the following manner. As the buttons are pushed together, the arms 63a and 63b are pushed together, moving the axle 65 of the control gear along slots 67 away from the drive gears 64, 66. The drive gears 64, 66, now engaged directly with each other, rotate in opposite directions with the result that the strips 42, 44 in teethed engagement with the gears 64, 66 both move in the same direction relative to the housing 54. This enables the housing 54 to be moved across the cranium, in line with the strips 42, 44 without allowing a change in tightness, or distance between the attachment ends of the strips.

In a tension adjustment position, as the strips 42, 44 move through the housing 54, they bias the drive gears 64, 66 together. In an embodiment, when the buttons 56 are not depressed, and are within a resting position outwardly biased, a releasable lock (not shown) is engaged to keep the arms 63a, 63bto which the drive gears 64, 66 are mounted 64, 66 from moving together until the buttons 56 are depressed. The button floats with respect to the arm so it can release the lock before pushing the arm 63a, 63b inwards. In one embodiment, there is a half-depressed state where drive and control gears are free to rotate independently so the holder can be easily moved, loosened or tightened without either arm 63a, 63b being affected by the other.

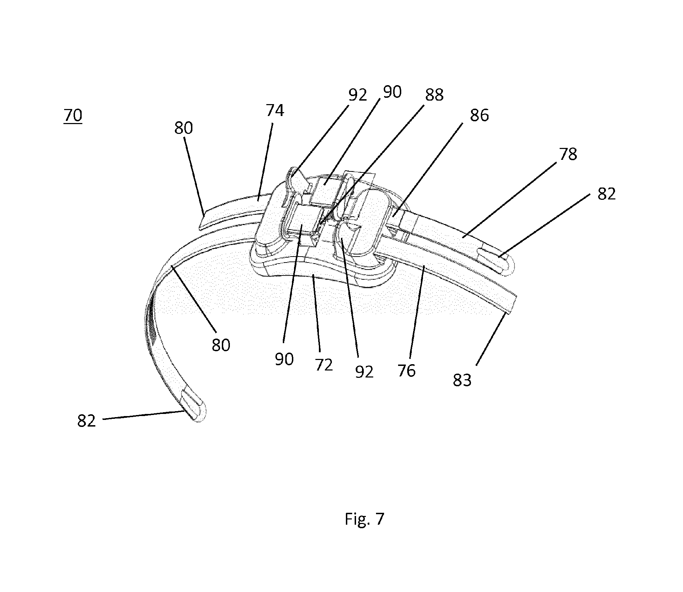

With reference to Figure 7, another embodiment of an electrode holder is described. The holder 70 has a housing 72 through which two tapes 74, 76 pass. A fastening end 78, 80 of each tape 74, 76 has a fastening means such as an eyelet 82 for attachment to the hooks 27 (not shown), wherein the opposite terminating end 83 simply terminates. The tapes 74, 76 are in opposite orientation to one another as they pass through the housing 72, wherein the fastening end 78 of tape 74 is on the same side of the housing 72 as the terminating end 83 of tape 76.The tapes 74, 76 have upwardly-facing teeth 86 thereon. On top of the housing, above each tape, is a releasable lock 88 operated by depression of a spring-biased button 90. The housing 72 may have two or more braces 92 extending therefrom to provide a brace for the fingers to push against when depressing button 90.

When the button 90 is depressed, the tape 74 or 76 slides freely through the lock 88. When the button 90 is released, the lock 88 locks the tape 74 or 76 into place and prevents further movement. The buttons 90 are in opposing orientation so that both buttons 90 may be squeezed simultaneously with one hand, in order to adjust the position of both tapes 74, 76 at the same time. Alternatively, a single button 90 may be depressed to adjust the position of the tape 74 or 76 below that lock 88 only.

## Claims

1. An electrode placement system, comprising:
a) a headband that is adjustable for size;
b) two or more retainers slidably mounted on the headband; and
c) a first elastic measurement cord,
wherein the measurement cord is releasably held by the two or more retainers in order to determine a position on a cranium.

2. The electrode placement system of claim 1, further comprising a second measurement cord affixed perpendicularly to the first measurement cord.

3. The electrode placement system of claim 1 or claim 2 wherein the measurement cord(s) has (have) distance markings thereon.

4. The electrode placement system of any of claims 1 to 3, further comprising flags affixed to the measurement cord, configured to measure a distance perpendicularly from the measurement cord.

5. The electrode placement system of claim 2 wherein the first measurement cord measures from a neck to a nose of a patient, and the second measurement cord measures from ear to ear of the patient.

6. The electrode measurement system of any preceding claim, wherein the retainers comprise:
a) a frame defining a restriction for a cord; and
b) a cam in rotating relation with the frame having a gripping edge
wherein when the cam is rotated in a tightening direction, the cam protrudes into the restriction, and wherein when the cam is rotated in a loosening direction, the cam recedes from the restriction, and wherein the gripping edge is configured to engage with the cord to rotate the cam.

7. An electrode retention system, comprising:
a) a headband that is adjustable for size, having a plurality of attachment points thereon;
b) two or more strips, each strip having two ends, wherein each strip is connected to an attachment point at one end; and
c) at least one adjustable electrode holder configured to hold an electrode to a cranium,
wherein first and second strips are connected to attachment points on the headband, and first and second strips pass through the at least one adjustable electrode holder, and the first and second strips are independently retained by the adjustable electrode holder such that the at least one electrode holder is adjustable for tension and position.

8. The electrode retention system of claim 7 wherein the headband is adjustable by an adjustment means, the adjustment means comprising:
toothed tabs extending from ends of the headband;
a rotatable gear in communication with the toothed tabs,
wherein when the gear is rotated in a tightening direction it actuates the toothed tabs in opposite directions, tightening the headband.

9. The electrode retention system of claim 7 or claim 8 wherein the attachment points comprise inverted hooks having apertures therebelow,
wherein the end of each strap has an eyelet, and the eyelet is configured to engage and disengage with the hook while the strap is in a horizontal orientation, and to be locked on the hook when the strap is in a vertical orientation.

10. The electrode retention system of any of claims 7 to 9 wherein the adjustable electrode holder comprises:
a) a housing through which the strips pass;
b) a gear cluster in the housing, comprising:
i) a control gear rotatably mounted on the housing by a displaceable axle;
ii) a first drive gear rotatably mounted to the housing and in communication with the first strip;
iii) a second drive gear rotatably mounted to the housing and in communication with the second strip,
wherein the strips have teeth for engaging with the drive gear teeth, and wherein in a tension adjustment position the first and second drive gears are connected by the control gear, and in a position adjustment position the control gear is displaced away from the drive gears, and the drive gears are connected directly to one another.

11. The electrode retention system of claim 10 further comprising a button on the housing for selecting a tension adjustment position and a position adjustment position, wherein the control gear is biased in a tension adjustment position.

12. The electrode retention system of any of claims 7 to 9 wherein the adjustable electrode holder comprises:
a housing through which the strips pass;
two releasable locks mounted to the housing, wherein a strip passes through each releasable lock;
a button for releasing each releasable lock;
wherein while the button is held the strip is movable within the releasable locks and when the button is released the strip is locked within the releasable lock.

13. The electrode retention system of claim 12 wherein each of the buttons for a releasable lock are opposite one another and are configured to be depressed by one hand.

14. The electrode retention system of claim 12 or claim 13 further comprising braces opposite the buttons for bracing to depress the button with one hand.
